# EUROPEAN PATENT APPLICATION

(11) **EP 2 044 891 A2**
(43) Date of publication of application: **08.04.2009**
(21) Application number: 08253192.2
(22) Date of filing: 30.09.2008
(51) Int. Cl.: A61B 17/068, A61B 17/16, A61C 1/18, B25B 17/00

(54) **Nutating gear mechanism for surgical devices**

(30) Priority: 05.10.2007 US 977708 P; 12.08.2008 US 189947
(71) Applicant: Tyco Healthcare Group LP, North Haven, CT 06473 (US)
(72) Inventor: Viola, Frank J., Sandy Hook CT 06482 (US)
(74) Representative: Soames, Candida Jane

(57) **Abstract**

A drive mechanism for transmitting rotation force in surgical devices comprises a nutating gear reduction drive (50) having an input (52) and an output (54). The input is configured to be driven at high speed, low torque by a proximal drive shaft (28) of a surgical device. The output is configured to transmit a low speed, high torque rotational force. The proximal drive shaft can be flexible. In one embodiment, the nutating gear reduction drive includes a wobble plate (60). This nutating gear reduction drive may include at least one crown gear. In an alternative embodiment, the nutating gear drive includes at least one ring gear. The ring gear is fixed in place. This embodiment can also include at least one spur gear.

## Description

### CROSS-REFERENCE TO RELATED APPLICATIONS

This application claims the benefit of and priority to U.S. Provisional Patent Application No. 60/977,708, filed October 5, 2007, the entire disclosure of which is incorporated by reference herein.

### BACKGROUND

### Technical Field

The present disclosure relates to drive mechanism for transmitting rotational force in surgical devices and, more particularly, to a nutating gear reduction drive for use with a surgical device.

### Background of Related Art

Surgeons often perform surgical procedures deep inside the human body. To facilitate such procedures, medical devices manufactures have developed numerous surgical instruments. These instruments or devices usually employ flexible shafts. Surgical devices use flexible shafts to transmit rotational forces from one point to another. Flexible shafts are particularly useful for surgical devices because they can easily bend and adjust their shape. This unique feature allows surgical devices with flexible shafts to easily navigate inside the human body.

Flexible shafts, however, tend to wind-up or twist when subject to high torque. Some surgical tools, which are often positioned at a distal end of a flexible shaft, require high torque to operate. To address this issue, engineers have developed surgical devices with flexible shafts that are configured to rotate at high speeds and low torque. The rotational forces of the flexible shafts are then converted or transformed into a low speed, high torque rotation by a speed reducing drive or mechanism.

Various speed reducing drives have been developed to transform high speed, low torque rotation into low seed rotation, high torque rotation. Surgical devices have employed some, but not all, speed reducing drives known in the art. So far, the speed reduction drives utilized in surgical devices have many moving parts and are therefore bulky.

For instance, some surgical devices use planetary gear assemblies to transform high speed, low torque rotation into low speed, high torque rotation. Typically, a planetary gear assembly has a centrally located sun gear. This sun gear is directly coupled to the drive shaft that provides the initial motive power. A set of gears, referred to as planet gears, are located around the sun gear. These planet gears are configured to mesh with the sun gear. A fixed ring surrounds the planetary gears. The inner surface of the ring has teeth. The teeth of the fixed ring gear are adapted to mesh with the planet gears. In addition, all the planet gears are connected to a common planet carrier. The planet carrier has a plurality of arms. Each arm is attached to a planet gear. As it is apparent from the foregoing description, planetary gear assemblies consist of many moving parts and, thus, are bulky.

Aside from planetary gear assemblies, certain surgical devices utilize spur gear trains as speed reduction drives. A single spur gear train can attain modest speed reduction rates. Generally, gear trains having multiple stages are necessary to achieve the high speed reduction rates required in surgical devices. Thus, speed reduction drives consisting of spur gears can have many moving parts and, as a consequence, can be bulky.

Nutating gear systems can also serve as speed reduction mechanisms.

### SUMMARY

The present disclosure relates to a drive mechanism for transmitting rotation forces in surgical devices. This mechanism comprises a nutating gear reduction drive having an input and an output. The input is configured to be driven at high speed, low torque by a proximal drive shaft of a surgical device. The proximal drive shaft can be flexible. The output is configured to transmit a low speed, high torque rotational force. The output can include a distal shaft. The distal shaft can be flexible. In one embodiment, the nutating gear reduction drive includes a wobble plate. Additionally, the nutating gear reduction drive may include at least one crown gear. In an alternative embodiment, the nutating gear drive has at least one ring gear. The ring gear is fixed in place. This nutating gear drive also includes at least one spur gear. The spur gear is configured to mesh with the ring gear. The proximal drive shaft includes a crank configured to rotate about a longitudinal axis. The crank has a pin extending distally. The pin is positioned in a location offset from the longitudinal axis.

Moreover, the present disclosure relates to surgical device having a drive mechanism for transmitting rotational forces. The drive mechanism includes a nutating gear reduction drive having an input and an output. The input is configured to be driven at high speed, low torque by a proximal drive shaft. The output is configured to transmit a low speed, high torque rotational force.

### DESCRIPTION OF THE DRAWINGS

An embodiment of the presently disclosed surgical device and drive mechanism for use therewith are disclosed herein with reference to the drawings, wherein:

FIG. 1 is a perspective view of a surgical device;

FIG. 2 is a perspective view of a portion of the surgical device of FIG. 1;

FIG. 3 is a sectional view of a drive mechanism of a surgical device in accordance with an embodiment of the present disclosure;

FIG. 4 is a perspective view of the drive mechanism of FIG. 3;

FIG. 5 is a side view of the drive mechanism of FIG. 3;

FIG. 6 is a perspective view a drive mechanism of a surgical device in accordance with an embodiment of the present disclosure;

FIG. 7 is a perspective view the drive mechanism of FIG. 6; and

FIG. 8 is a perspective cross-sectional view of the drive mechanism of FIG. 6.

### DETAILED DESCRIPTION OF THE EMBODIMENTS

Embodiments of the presently disclosed surgical devices and drive mechanisms are described in detail with reference to the drawings, in which like reference numerals designate identical or corresponding elements in each of the several views. In the drawings and in the description that follows, the term "proximal", as is traditional, will refer to the end of surgical device, or portion thereof, that is closest to the operator while the term "distal" will refer to the end of the device, or portion thereof, that is farthest from the operator. Also, as used herein, all singular forms, such as "a," "an," and "the," are intended to include the plural forms as well, unless expressly stated otherwise.

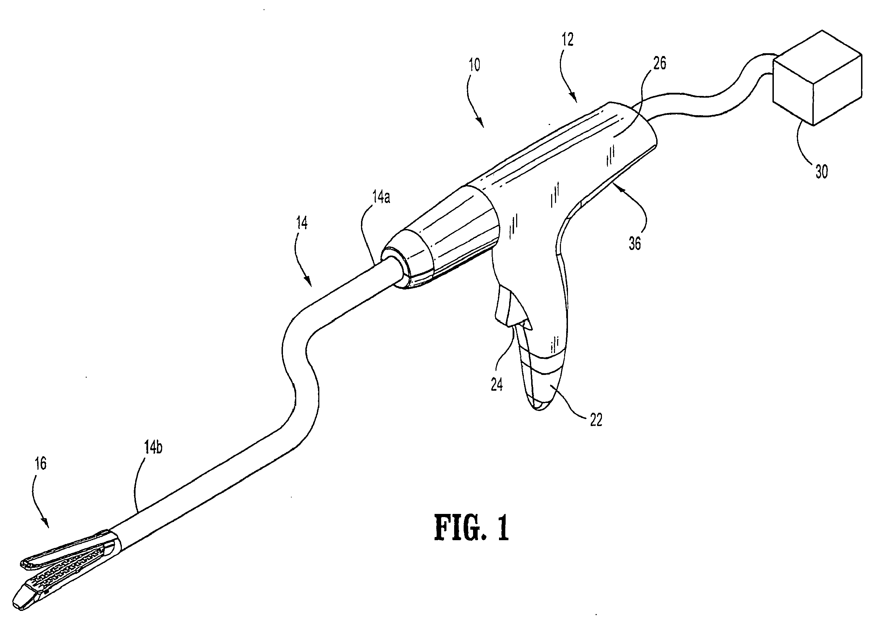

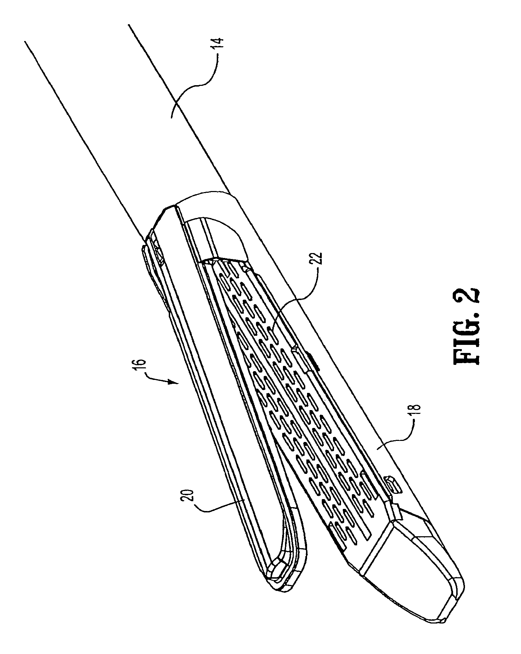

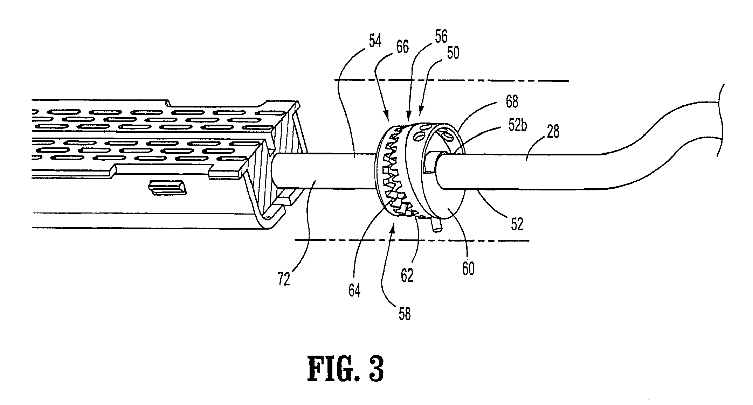

Referring initially to FIGS. 1 and 2, a surgical device is generally designated as reference numeral 10. Although the drawings depict surgical device 10 as a surgical stapling apparatus, the present disclosure contemplates other suitable medical devices. Briefly, the illustrated surgical device 10 includes a handle assembly 12, an elongated body 14, and a surgical tool 16. Handle assembly 12, which is operatively coupled to elongated body 12, includes a handle member 22, a button 24, and a barrel portion 26. In addition, handle assembly 12 has a housing 36. A motor or any other suitable driving mechanism can be positioned inside the housing 36. Alternatively, a motor 30 can disposed outside handle assembly 12 and in electromechanical cooperation with surgical device 10, as shown in FIG. 1. Regardless of its location, motor 30 is operatively connected to a drive shaft 28 (see FIG. 3). Users can activate motor 30 by pressing button 24 of handle assembly 12. Thus, button 24 is adapted to start motor 30. Since motor 30 is operatively connected to drive shaft 28, the activation of motor 30 causes the rotation of drive shaft 28. In particular, motor 30 is configured to rotate drive shaft 30 at high speed, low torque. Drive shaft 28, in turn, extends from handle assembly 12 through elongated body 14.

Elongated body 14 encompasses at least a portion of drive shaft 28. A proximal end 14a of elongated body 14 is operatively coupled to handle assembly 12. A distal end 14b of elongated body 14, in turn, is operatively secured to surgical tool 16. Elongated body 14 can be made of a flexible material. In use, a flexible elongated body 14 allows surgeons to easily guide surgical tool 16 to a desired surgical site.

Surgical tool 16 is attached to the distal end 14b of elongated body 14 and includes a cartridge assembly 18 and an anvil assembly 20. Anvil assembly 20 is movably secured in relation to cartridge assembly 18. Cartridge assembly 18 has retention slots 22. Retention slots 22 are adapted to receive surgical fasteners. In the drawings, retention slots 22 are arranged in linear rows. The present disclosure, however, envisions retentions slots 22 arranged in any suitable manner. Altogether, surgical tool 16 is configured to apply surgical fasteners to a tissue portion. It is contemplated that surgical tool 16 can be an end effector or any other suitable surgical instrument.

As discussed above, surgical device 10 can include a flexible elongated body 14. Elongated body 14, however, can also be rigid. Surgical devices 10 with a rigid elongated body 14 can include an articulation mechanism to articulate surgical tool 16. The articulation mechanism includes an articulation level. The articulation level can be mounted on the distal end of barrel portion 26 to facilitate articulation of surgical tool 16.

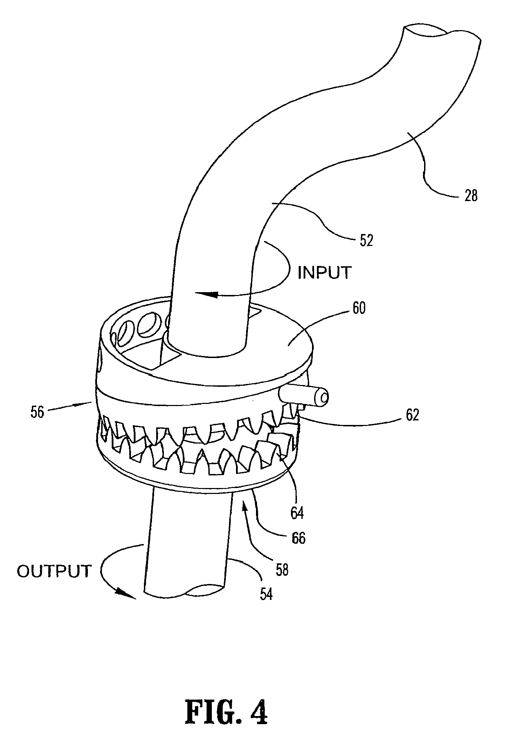

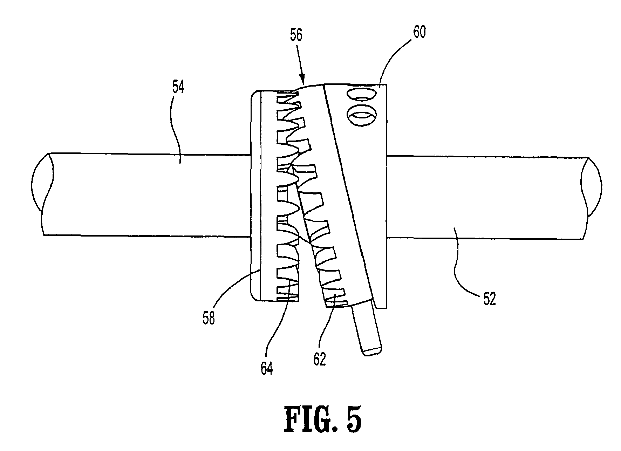

With reference to FIGS. 3-5, the depicted surgical device 10 includes a flexible drive shaft 28 and a flexible elongated body 14. Drive shaft 28 is disposed in a proximal location with respect to nutating gear reduction drive 50. Nutating gear reduction drive 50 is operatively connected to drive shaft 28 and is configured to transmit rotational forces from drive shaft 28. In particular, nutating gear reduction drive 50 transforms the high speed, low torque rotational force of drive shaft 28 into a low speed, high torque rotational force. The high speed, low torque rotation force delivered by nutating gear drive 50 is capable of actuating surgical tool 16 or any other suitable medical tool.

Nutating gear reduction drive 50 includes an input 52 and an output 54. Input 52 is configured to be driven by a drive shaft 28 at high speed, low torque. Thus, input 52 is operatively connected to the drive shaft 28. Input 52 includes a pressing member 68 positioned at its distal end 52b. Pressing member 68 is adapted to press and incline at least a portion of a first gear 56. A crank, a rotor, or any other suitable apparatus can be used as a pressing member 68. Irrespective of the specific apparatus employed, pressing member 68 should be capable of inclining and rotating first gear 56. During use, the inclined rotation of first gear 56 rotates a second gear 58.

As discussed above, nutating gear drive 50 includes a first gear 56 and a second gear 58. First gear 56 has a wobble plate 60 disposed in mechanical cooperation with input 52. Wobble plate 60 can be a crown gear or any other suitable gear. In addition to the wobble plate 60, first gear 56 includes teeth 62. Teeth 62 face second gear 58 and are configured to mesh with teeth 64 of second gear 58. In operation, pressing member 68 inclines first gear 56 so that only some teeth 62 of first gear 56 mesh with teeth 64 of second gear 58. Pressing member 68 also causes first gear 56 to wobble as input 52 rotates at high speed and low torque.

Second gear 58 includes a plate 66. Although plate 66 is not configured to wobble, it is adapted to rotate in response to the rotation wobble plate 60. Additionally, second gear 58 includes teeth 64. As discussed above, teeth 64 of second gear 58 are configured to mesh with teeth 62 of first gear 56. In one embodiment, first gear 56 has first predetermined number of teeth 62 that is different from a second predetermined number of teeth 64 of second gear 58. The speed reduction ratio of nutating gear reduction drive 50 is dictated by the difference in the number of teeth between first gear 56 and second gear 58. Also, nutating gear reduction drive 50 may include multiple stages to produce even higher speed reduction ratios.

Irrespective of the number of stages, nutating gear reduction drive 50 includes an output 54 configured to rotate at low speed, high torque. Output 54 transmits its rotational forces to a distal shaft 72. Distal shaft 72 can be flexible. In any case, distal shaft 72 is disposed in mechanical cooperation with surgical tool 16. It is the rotation of distal shaft 72 that causes the actuation of surgical tool 16.

During operation, a surgeon initially presses button 24 to activate a motor 30 to rotate drive shaft 28 at high speed, low torque. As drive shaft 28 rotates, input 52 rotates along with its pressing member 68. The rotation of input 52 causes the rotation and wobbling of first gear 56. As first gear 56 rotates and wobbles, only some teeth 62 of first gear 56 mesh with teeth 64 of second gear 58. The difference in the number of teeth between first gear 56 and second gear 58 dictates the speed reduction ratio. Specifically, when first gear 56 effects one full rotation, second gear 58, which is only partially meshing with first gear 56, rotates by an amount corresponding to the difference in the number of teeth between first gear 56 and second gear 58.

While first gear 56 wobbles and rotates, second gear 58 rotates, thereby causing output 54 to rotate at low speed, high torque. The low speed, high torque rotation of output 54 effectively actuates surgical tool 16. In the depicted embodiment, when surgical tool 16 is actuated, anvil assembly 20 moves and approximates cartridge assembly 18 to clamp tissue. Also, the surgical fasteners retained in retentions slots 22 deploy and fasten tissue portions together. Nevertheless, as discussed above, any suitable surgical instrument can be employed with nutating gear reduction drive 50.

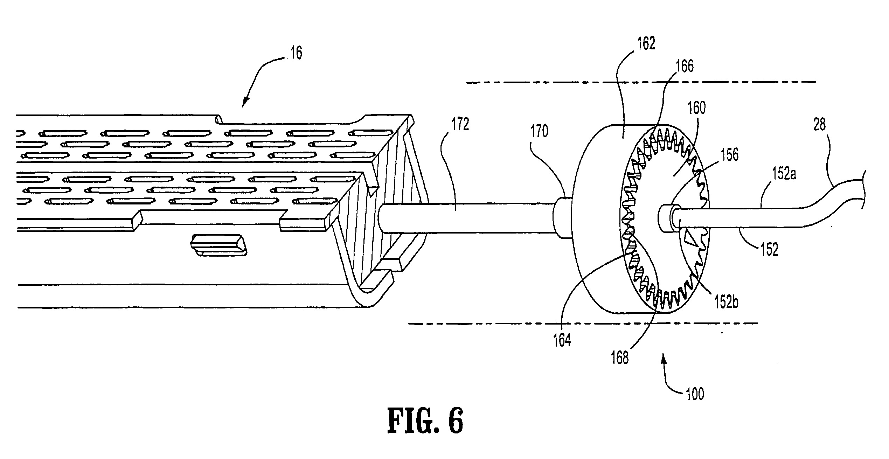

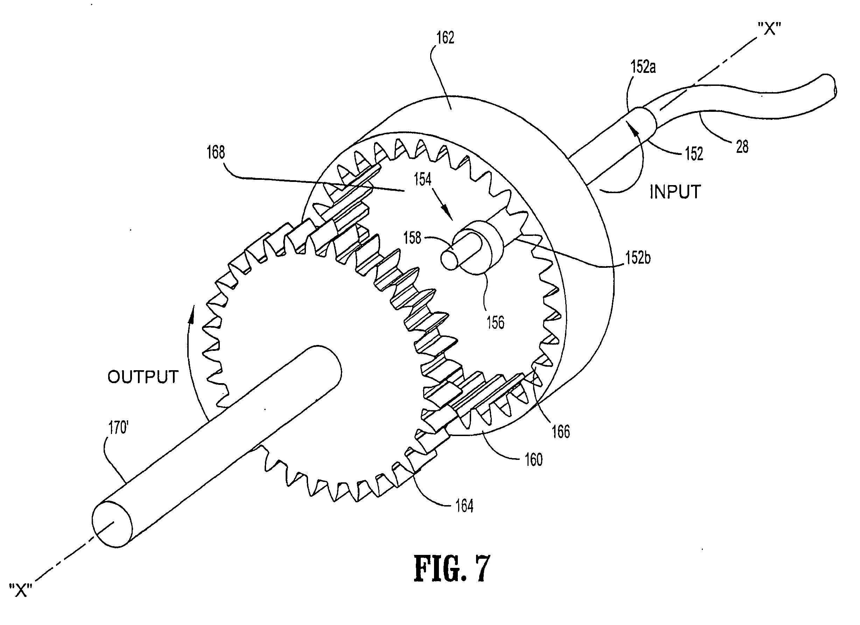

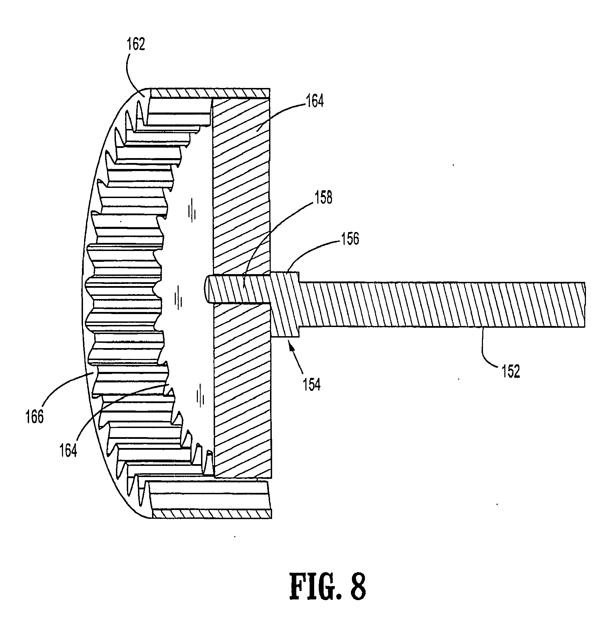

Referring to FIGS. 6-8, in an alternative embodiment, a nutating gear reduction drive 100 has an input 152 and an output 170. Nutating gear reduction drive 100 is configured to transmit rotational forces from input 152 to output 170. In particular, nutating gear reduction drive 100 transforms the high speed, low torque rotational force of input 152 into a low speed, high torque rotational force.

Input 152 of nutating gear drive 100 includes a proximal end 152a and a distal end 152b. A crank 154 is disposed on the distal end 152b of input 152 and includes a tubular member 156 and a pin 158. Tubular member 156 defines a longitudinal axis "X" and pin 158 extends distally from a location offset from the longitudinal axis "X." Altogether, crank 156 is operatively connected to a first gear 160. First gear 160 can be a spur gear or any other suitable kind of gear. First gear 160 has teeth 164 that extend radially and outwardly. In operation, first gear 160 rotates about its center and about the center of a second gear 162.

Nutating gear drive 100 also includes a second gear 162. Second gear 162 is fixed in place and has a bore 168 extending therethrough. In one embodiment, second gear 162 is secured to elongated body 14 by a fastening member. Bore 168 is adapted to receive first gear 160. Second gear 162 can be a ring gear or any other suitable gear. In addition, second gear 162 includes teeth 166. Teeth 166 of second gear 162 extend radially and inwardly towards bore 168. Moreover, teeth 166 of second gear 162 are configured to mesh with teeth 164 of first gear 160. During operation, only some teeth 164 of first gear 160 mesh with teeth 166 of second gear 162.

As discussed above, nutating gear reduction drive 100 includes an output 170 operatively coupled to first gear 164. Although the drawings show an output 170 having a cylindrical shape, output 170 may have any suitable shape. Further, output 170 can be operatively connected to a distal shaft 172. Distal shaft 172 can be made of a flexible material. As shown in FIG. 6, distal shaft 172 is operatively connected to surgical tool 16. During operation, the low speed, high torque rotation of distal shaft 172 actuates surgical tool 16. In the depicted embodiment, the rotation of distal shaft 172 causes the movement of anvil assembly 20. Specifically, anvil assembly 20 moves in relation to cartridge assembly 18 to clasp tissue. Further, the fasteners disposed in retention slots 22 of cartridge assembly 18 deploy in response to the rotation of distal shaft 172.

To actuate surgical tool 16, a user initially presses button 24 to start a motor 30. Motor 30, in turn, rotates drive shaft 28 at high speed, low torque. The rotation of drive shaft 28 causes input 152 to rotate, thereby rotating crank 154. As crank 154 rotates, first gear 160 rotates about its center and about the center of second gear 162. During this rotation, some, but not all, of teeth 164 of first gear 160 mesh with teeth 166 of second gear 162. Second gear 162 is fixed in place and does not move in response to the rotation of first gear 160. The rotation of first gear 160, however, causes the rotation of output 170. Due to the interaction between first gear 160 and second gear 162, output 170 rotates at low speed and high torque. Output 170 then transmits its rotational forces to distal shaft 172 to actuate surgical tool 16. The rotation of distal shaft 172 provides the torque necessary to actuate surgical tool 16.

Surgical tool 16 can be an end effector, as depicted in FIG. 1, or any other suitable surgical instrument. During operation, the surgical tool 16 of the illustrated embodiment actuates in response to the rotation of output 170. Specifically, the rotation of output 170 causes the movement of anvil assembly 20. In particular, anvil assembly 20 moves in relation to cartridge assembly 18 to clasp tissue. Additionally, the rotation of output 170 deploys the fasteners disposed in retention slots 22 of cartridge assembly 18.

It will be understood that various modifications may be made to the embodiments disclosed herein. Therefore, the above description should not be construed as limiting, but merely as exemplifications of embodiments. Those skilled in the art will envision other modifications within the scope and spirit of the claims appended thereto.

A drive mechanism for transmitting rotation force in surgical devices comprises a nutating gear reduction drive having an input and an output. The input is configured to be driven at high speed, low torque by a proximal drive shaft of a surgical device. The output is configured to transmit a low speed, high torque rotational force. The proximal drive shaft can be flexible. In one embodiment, the nutating gear reduction drive includes a wobble plate. This nutating gear reduction drive may include at least one crown gear. In an alternative embodiment, the nutating gear drive includes at least one ring gear. The ring gear is fixed in place. This embodiment can also include at least one spur gear.

## Claims

1. A drive mechanism for transmitting rotational force in surgical devices, comprising:
a nutating gear reduction drive having an input and an output , the input configured to be driven at high speed, low torque by a proximal drive shaft of a surgical device and the output configured to transmit a low speed, high torque rotational force.

2. The drive mechanism of claim 1, wherein the proximal drive shaft is flexible.

3. The drive mechanism of claim 1, wherein the output comprises a distal shaft.

4. The drive mechanism of claim 3, wherein the distal shaft is flexible.

5. The drive mechanism of claim 1, wherein the nutating drive gear reduction drive includes a wobble plate.

6. The drive mechanism of claim 1, wherein nutating drive gear reduction drive includes at least one crown gear.

7. The drive mechanism of claim 1, where the nutating drive gear reduction drive includes at least one ring gear.

8. The drive mechanism of claim 7, wherein the ring gear is fixed in place.

9. The drive mechanism of claim 7, wherein the nutating drive gear reduction drive includes at least one spur gear.

10. The drive mechanism of claim 9, wherein the at least one spur gear is configured to mesh with the ring gear.

11. The drive mechanism of claim 1, wherein the proximal drive shaft includes a crank configured to rotate about a longitudinal axis.

12. The drive mechanism of claim 11, wherein the crank includes a pin extending distally.

13. The drive mechanism of claim 10, wherein the pin is positioned in a location offset from the longitudinal axis.

14. A surgical device, comprising:
a drive mechanism for transmitting rotational force, including:
a nutating gear reduction drive having an input and an output, the input configured to be driven at high speed, low torque by a proximal drive shaft of a surgical device and the output configured to transmit a low speed high torque rotational force.

15. The surgical device of claim 14, wherein the proximal drive shaft is flexible.

16. The surgical device of claim 14, wherein the output comprises a distal shaft.

17. The surgical device of claim 16, wherein the distal shaft is flexible.

18. The surgical device of claim 14, wherein the nutating gear reduction drive includes a wobble plate.

19. The surgical device of claim 14, wherein the nutating gear reduction drive includes at least one ring gear.

20. The surgical device of claim 14, wherein the nutating gear reduction drive includes at least one spur gear.
